# EUROPEAN PATENT APPLICATION

(11) **EP 4 692 764 A1**
(43) Date of publication of application: **11.02.2026**
(21) Application number: 25194530.9
(22) Date of filing: 07.08.2025
(51) Int. Cl.: G01N 21/25, G01N 21/33, G01N 21/88, G01N 21/89, G01N 21/898, G01N 33/46, G01N 21/27, G01N 21/892, G01N 21/17, G01N 21/84

(54) **A SYSTEM AND A METHOD FOR DETECTING DEFECTS IN WOOD**

(30) Priority: 08.08.2024 PL 44948124
(71) Applicant: Centrum Badan i Rozwoju Technologii dla Przemyslu S.A., 00-645 Warszawa (PL)
(72) Inventor: KLEMIATO, Maciej, 00-645 Warszawa (PL); KNAPIK, Dawid, 00-645 Warszawa (PL); PUTYNKOWSKI, Grzegorz, 00-645 Warszawa (PL); ROSOL, Maciej, 00-645 Warszawa (PL); ROTTER, Pawel, 00-645 Warszawa (PL); ZBROJA, Krzysztof, 00-645 Warszawa (PL); KASZA, Patryk, 00-645 Warszawa (PL)
(74) Representative: Kancelaria Eupatent.pl Sp. z o.o.

(57) **Abstract**

A system for detecting defects in wood, comprising: an inspection station (10) configured to receive a wood workpiece; a light source (21) configured to illuminate the wood workpiece at the inspection station (10) with light having a wavelength in the range of 225 nm to 625 nm; an imaging device (22) configured to measure the absorbance of the light by the illuminated area of the wood workpiece; and an analyzer (23) configured to classify areas exhibiting absorbance above a predetermined threshold as areas containing a resin defect.

## Description

### TECHNICAL FIELD

The present invention relates to a system and a method for detecting defects in wood, in particular in wood intended for furniture, construction, artistic, transport, or instrument use.

### BACKGROUND

In the woodworking industry, particularly in sectors involving furniture manufacturing, construction, artistic applications, transport, and musical instrument production, ensuring the quality of wood material is of significant importance. High-quality wood is typically understood to mean wood substantially free of defects such as knots, resin pockets, voids, and discolorations. Such defects not only compromise aesthetic appeal but may also lead to material weakness, reducing the reliability and lifespan of finished wood products. Additionally, industry standards such as Polish Standard PN-79/D-01011 explicitly define wood defects as visible structural or color anomalies.

Various techniques for detecting defects in wood by means of optical imaging and subsequent image analysis are known from prior art.

For example, Canadian patent publication CA3054959 describes a method employing machine learning, specifically a convolutional neural network trained for semantic segmentation. This approach analyses image pixels to classify wood characteristics, thus facilitating improved accuracy and efficiency in wood quality control.

Chinese patent publication CN107392896 discloses an image-based defect detection system which involves segmenting captured wood images into blocks, classifying these blocks into defective and non-defective groups, and utilizing deep learning techniques to accelerate defect detection.

Further, Chinese patent publication CN115294113 describes assessing veneer quality by segmenting images into superpixel blocks, analyzing characteristics such as color saturation and grey-scale values, enabling a detailed evaluation of veneer quality.

Additionally, Chinese patent publication CN110310259 teaches an advanced YOLOv3-based algorithm for detecting knot defects. The described system incorporates image data augmentation and clustering via k-means++ algorithms to enhance detection performance.

### SUMMARY OF THE INVENTION

Despite the prior art developments, there remains an ongoing need for alternative or improved non-destructive approaches to reliably and efficiently detect defects in wood, particularly methods which are readily integrable into manufacturing processes without significantly disrupting existing production workflows.

The aim of the present invention is to develop an alternative non-destructive method for detecting defects in wood, such as knots, voids, and resin pockets.

The inventors of the present invention have made a previously unknown observation that knots and resin pockets show greater absorbance of light in the range of 220-620 nm, in particular in the UV range (225-355 nm) and in the visible range (380-620 nm), compared to wood samples without such defects. This allowed the inventors to develop a system and method for detecting resin defects in wood as described below.

In one aspect of the invention, a system for detecting defects in wood is provided, comprising an inspection station configured to receive a wood workpiece, a light source configured to illuminate the wood workpiece at the inspection station with light having a wavelength in the range of 225 nm to 625 nm, an imaging device configured to measure the absorbance of the light by the illuminated area of the wood workpiece, and an analyzer configured to classify areas exhibiting absorbance above a predetermined threshold as areas containing a resin defect. Such configuration provides rapid and accurate detection of defects, enabling consistent quality control without destructive testing.

In a preferred embodiment, the inspection station comprises at least one of a conveyor belt, rotary table, linear platform, or holder assembly for fixing wood workpieces. This allows flexible integration into various manufacturing setups and enhances automated handling efficiency.

In another preferred embodiment, the light source comprises at least one of a diode, laser, xenon lamp, LED lamp, halogen lamp, fluorescent lamp, mercury lamp, or deuterium lamp. Therefore, it is possible to select a suitable light source matching specific inspection requirements, optimizing sensitivity and detection accuracy.

In another preferred embodiment, the wavelength of the light source is in the ultraviolet or visible range and is monochromatic or has a defined spectral bandwidth. This ensures optimal absorption contrast between defective and defect-free wood areas, significantly improving detection precision.

Another preferred embodiment includes an imaging device comprising at least one of a monochromatic spectrometer, array-detector spectrometer, optical profilometer imaging device, monochromatic camera, RGB camera, or multispectral camera. This configuration allows for detailed analysis of wood surfaces, enhancing the capability to detect subtle defects.

In another preferred embodiment, the imaging device includes an optical filter configured to transmit a specific wavelength range. Such a setup enhances the selectivity of defect detection, increasing the accuracy of identifying resin defects specifically.

In another preferred embodiment, the light source and imaging device are integrated into a single device, preferably an optical profilometer. This simplifies the overall system configuration, reducing complexity and installation space requirements.

Another preferred embodiment provides that the inspection station moves the wood workpiece relative to the light source and imaging device. Therefore, it is possible to continuously scan and efficiently inspect larger or multiple wood workpieces.

Another preferred embodiment comprises a configuration wherein the light source and imaging device are movable relative to the inspection station. This flexibility allows detailed scanning and precise inspection of complex or large wood surfaces.

In another aspect of the invention, a method for detecting defects in wood is disclosed, including providing a wood workpiece to an inspection station, illuminating an area of the wood workpiece with light in the range of 225 nm to 625 nm, measuring absorbance of the illuminated area, and classifying areas with absorbance above a predetermined threshold as resin defects. This allows for non-destructive, rapid, and precise detection of wood defects, thus increasing the quality and reliability of wood products.

In a preferred embodiment, the method further comprises calibrating the system by measuring absorbance from defect-free wood workpieces to establish baseline values, with sensitivity adjusted such that intensity values at defect locations approach zero. This ensures accurate differentiation between defect-free and defective wood, thereby improving detection consistency.

Another preferred embodiment includes classifying resin defects using a rule-based classifier with predefined threshold values. This allows straightforward implementation and transparent criteria for defect identification.

Alternatively, resin defects may be classified using an artificial neural network trained specifically for this task. Such configuration provides advanced detection capabilities, including adaptive learning and improved accuracy in varied wood conditions.

Another preferred embodiment includes outputting classification parameters such as defect presence, number, size, location, and defect type. This enables detailed quality analysis and supports efficient sorting and processing decisions.

In another preferred embodiment, absorbance measurements are performed using an optical profilometer, and classification is based on both surface profile data and absorbance values. This combined approach significantly enhances detection accuracy and enables precise identification and classification of various defect types.

A skilled person will realize that the features of the preferred embodiments may be combined to form further preferred embodiments, depending on the expected capabilities of the method or system.

These and other features, aspects and advantages of the invention will become better understood with reference to the following drawings, descriptions and claims.

### BRIEF DESCRIPTION OF DRAWINGS

The invention is shown by means of example embodiments in a drawing, wherein:
Fig. 1 shows an example embodiment of a system for detecting defects in wood;
Fig. 2 shows example images obtained during detection.

### DETAILED DESCRIPTION

The following detailed description is one of preferable modes of carrying out the invention. The description is not to be taken in a limiting sense, but is made merely for the purpose of illustrating the general principles of the invention.

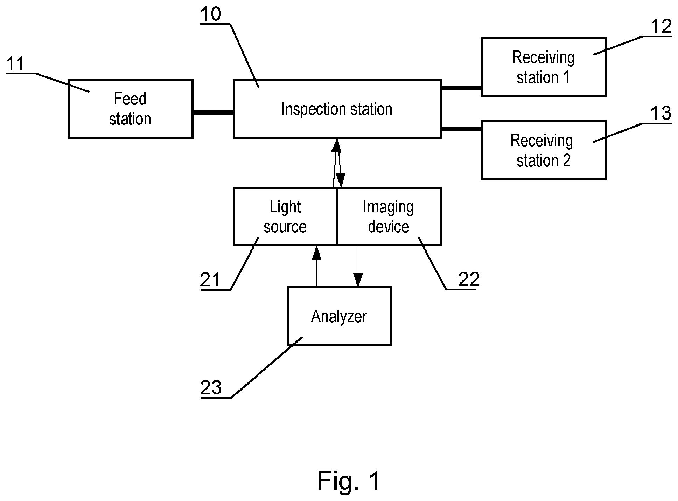

With reference to Figure 1, an embodiment of the invention is illustrated, showing schematically a system configured to detect defects in wood. The system comprises an inspection station 10 arranged to convey wood workpieces from a feed station 11 toward one of two distinct receiving stations, a first receiving station 12 for defect-free workpieces and a second receiving station 13 for workpieces containing defects. The transport of workpieces between these stations 10, 11, 12, 13 may be performed manually or through automated handling devices.

Positioned above the inspection station 10 is an optical profilometer which incorporates a light source 21, such as a 405 nm laser, and an imaging device 22, such as a camera, configured to capture reflected light from the illuminated wood surface. The optical profilometer generates an image (bitmap), wherein each pixel corresponds to measured intensity and optionally depth information, indicative of the structural characteristics and reflectivity of the inspected surface of the wood workpiece. The imaging device 22 transmits acquired image data to an analyzer unit 23.

In a preferred embodiment, the analyzer 23 processes intensity data of the reflected light obtained from the imaging device 22 to determine the absorbance characteristics of the inspected wood surface. Alternatively, more sophisticated processing may involve both intensity and depth data to achieve comprehensive defect detection.

The analyzer 23 is initially calibrated using reference measurements obtained from known defect-free wood samples. Specifically, calibration involves recording reflectance spectra from representative defect-free wood workpieces (in particular, of the same type that is going to be inspected following the calibration), thereby establishing a baseline intensity range (and thus, indirectly, the absorbance of light by the wood). For instance, using an 8-bit scale (values 0 to 255), the sensitivity of the imaging device 22 is adjusted such that defect-free areas yield intensity values significantly above zero, for example within a predetermined range of approximately 51-255 (i.e. for each pixel of the image representing wood without defect, the expected brightness will be in the range from 51 to 255). Conversely, areas corresponding to defects, particularly resin pockets, exhibit markedly lower reflectance due to increased absorption at the selected wavelength, resulting in intensity values approaching or equaling zero. Consequently, defects become readily distinguishable from defect-free areas by virtue of their distinctively lower intensity readings. In other words, the sensitivity of the imaging device is set so as to reproduce as accurately as possible the differences in intensity of areas of wood without defects (i.e., the upper limit of the range corresponds to the highest expected intensity of light reflected from wood without defect), and to cut off areas with defects (i.e., the lower limit of the range corresponds to the highest expected intensity of light reflected from wood with resin defect).

The analyzer 23 employs an image analysis algorithm which may include a rule-based classifier applying predefined threshold values or, alternatively, an artificial neural network suitably trained to identify defect-related areas. Based on predefined thresholds, detected areas can be categorized according to their size and intensity, for instance: small defects below a certain threshold may be disregarded, intermediate defects falling between defined thresholds classified accordingly, and large defects exceeding upper thresholds distinctly identified. The analyzer 23 is thus capable of providing detailed information regarding the number, size, position, and potentially the nature of detected defects. Analysis of depth information may further differentiate defect types such as voids (depressed areas), resin pockets (protruding areas), or flat resin-related anomalies.

Following analysis, wood workpieces are sorted accordingly and directed either to the first receiving station 12 (defect-free wood) or to the second receiving station 13 (wood containing defects), as determined by the analyzer 23.

### Operation example

In an exemplary embodiment, pine wood strips having dimensions of 48 mm x 16 mm x 750 mm were inspected. The optical profilometer, comprising the light source 21 and imaging device 22, was installed approximately 360 mm above the surface of a conveyor belt constituting inspection station 10. A monochromatic laser beam at a wavelength of 405 nm was projected perpendicularly onto the wood surface. Each profile image acquired by the profilometer comprised approximately 3200 measurement points along the width of the workpiece. The conveyor belt 10 ensured continuous relative movement between the profilometer and the wood workpieces, enabling sequential capture of multiple profiles and thus forming a comprehensive three-dimensional dataset.

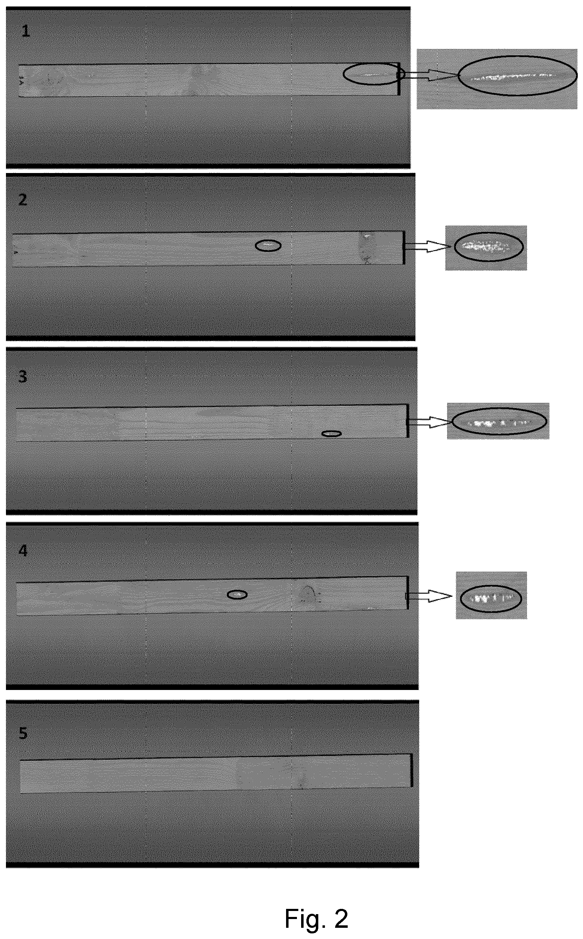

Ultimately, height maps consisting of approximately 3200 x 8000 points per wood strip were generated, each data point incorporating intensity measurements of reflected light. Figure 2 illustrates representative images with indicated defect locations (enlarged), specifically identifying areas exhibiting near-total absorption of laser illumination (as exemplified by the first four images), thus classified as resin pockets. In contrast, the fifth image represents a defect-free wood sample, demonstrating uniform intensity levels consistent with established calibration thresholds.

While the invention has been described with respect to a limited number of embodiments, it will be appreciated that many variations, modifications and other applications of the invention may be made. Therefore, the claimed invention as recited in the claims that follow is not limited to the embodiments described herein.

## Claims

1. A system for detecting defects in wood, comprising:
- an inspection station (10) configured to receive a wood workpiece;
- a light source (21) configured to illuminate the wood workpiece at the inspection station (10) with light having a wavelength in the range of 225 nm to 625 nm;
- an imaging device (22) configured to measure the absorbance of the light by the illuminated area of the wood workpiece; and
- an analyzer (23) configured to classify areas exhibiting absorbance above a predetermined threshold as areas containing a resin defect.

2. The system according to claim 1, wherein the inspection station (10) comprises at least one of a conveyor belt, a rotary table, a linear platform, and a holder assembly for fixing wood workpieces.

3. The system according to claim 1 or 2, wherein the light source (21) comprises at least one of a diode, a laser, a xenon lamp, a LED lamp, a halogen lamp, a fluorescent lamp, a mercury lamp, and a deuterium lamp.

4. The system according to any one of previous claims, wherein the wavelength of the light source (21) is in the ultraviolet (UV) or visible range and wherein the light emitted by the light source (21) is monochromatic or has a defined spectral bandwidth.

5. The system according to any one of previous claims, wherein the imaging device (22) comprises at least one of a monochromatic spectrometer, an array-detector spectrometer, an optical profilometer imaging device, a monochromatic camera, an RGB camera, and a multispectral camera.

6. The system according to any one of previous claims, wherein the imaging device (22) comprises an optical filter configured to transmit a specific wavelength range.

7. The system according to any one of previous claims, wherein the light source (21) and the imaging device (22) are integrated into a single device, preferably an optical profilometer.

8. The system according to any one of previous claims, wherein the inspection station (10) is configured to move the wood workpiece relative to the light source (21) and the imaging device (22).

9. The system according to any one of previous claims, wherein the light source (21) and the imaging device (22) are movable relative to the inspection station (10).

10. A method for detecting defects in wood, comprising:
- providing a wood workpiece to an inspection station (10);
- illuminating, by means of a light source (21), an area of the wood workpiece with light having a wavelength in the range of 225 nm to 625 nm;
- measuring, by means of an imaging device (22), absorbance of the light by the illuminated area of the wood workpiece;
- classifying, by means of an analyzer (23), areas with absorbance above a predetermined threshold as areas containing a resin defect.

11. The method according to claim 10, further comprising calibrating the system by measuring the absorbance of light from defect-free wood workpieces to determine baseline absorbance values, and wherein the calibration preferably comprises adjusting the sensitivity range of the imaging device (22) such that intensity values at defect locations approach zero.

12. The method according to any one of claims 10 to 11, wherein classifying areas containing a resin defect comprises applying a rule-based classifier utilizing predefined threshold values.

13. The method according to any one of claims 10 to 12, wherein classifying areas containing a resin defect comprises applying an artificial neural network trained to recognize resin defects.

14. The method according to any one of claims 10 to 13, further comprising outputting at least one classification parameter selected from: an indication of the presence of areas containing a resin defect; the number of areas containing a resin defect; the size of each area containing a resin defect; the location of each area containing a resin defect; and the type of each detected defect.

15. The method according to any one of claims 10 to 14, wherein measuring absorbance is performed using an optical profilometer, and classification is based on both surface profile data and measured absorbance values.
